(19) Europäisches Patentamt / European Patent Office / Office européen des brevets

(11) **EP 1 982 603 A1**

(12) **EUROPEAN PATENT APPLICATION**

(43) Date of publication:
**22.10.2008 Bulletin 2008/43**

(21) Application number: **07007874.6**

(22) Date of filing: **18.04.2007**

(51) Int Cl.:
*A23L 1/30* (2006.01)  *A61K 31/05* (2006.01)
*A61K 31/7048* (2006.01)  *A23L 2/52* (2006.01)

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(71) Applicant: **DSM IP Assets B.V.**
**6411 TE Heerlen (NL)**

(72) Inventor: **The designation of the inventor has not yet been filed**

(74) Representative: **Steck, Melanie et al**
**DSM Nutritional Products Ltd**
**Wurmisweg 576**
**CH-4303 Kaiseraugst (CH)**

(54) **Novel use of hydroxytyrosol and olive extracts/concentrates containing it**

(57) The present invention is directed to the use of (a composition comprising) hydroxytyrosol as life prolonging agent. The composition, to which the present invention is also directed, does essentially not comprise resveratrol and is preferably administered orally to animals.

The present invention is further directed to a life prolonging method. "Life prolonging" meaning in the context of the present invention:
reducing the risk to die at a certain age compared to untreated;
increasing the chance to live longer (longevity), i.e. increasing the average life expectancy compared to untreated; and/or
extending the lifetime independent of medical care.

EP 1 982 603 A1

**Description**

The present invention is directed to the use of (a composition comprising) hydroxytyrosol as life prolonging agent. The composition, to which the present invention is also directed, does essentially not comprise resveratrol and is administered orally to animals. The present invention is further directed to a life-prolonging method. "Life prolonging" meaning in the context of the present invention:

reducing the risk to die at a certain age compared to untreated;
increasing the chance to live longer (longevity), i.e. increasing the average life expectancy compared to untreated; and/or
extending the lifetime independent of medical care.

Thus, hydroxytyrosol is capable of making you live longer; prolonging your life; letting you get older happily and/or expending your lifespan.

Longevity is the length of a person's life (life expectancy). Various factors contribute to an individual's longevity. Significant factors in life expectancy include gender, genetics, access to health care, hygiene, diet, exercise, lifestyle, and crime rates.

Reaching an old age has fascinated people for ages. There are many organizations dedicated to exploring the causes behind aging, ways to prevent aging, and ways to reverse aging. This shows that there is a great need for means to increase the chances to live longer.

From the American Journal of Clinical Nutrition 2006 (Feb), 83(2), 484S-487S: "The different paths to 100. Perls TT." the following is known:

Approximately 90% of centenarians in a population-based study were functionally independent at the average age of 92 years. Thus, to achieve extreme old age, a much more enabling point of view emerges: the older an individual gets, the healthier he or she has been. Centenarians thus have the potential to represent a model of relative resistance to age-related diseases and slower aging. Currently, 1 in every 10 000 persons in the United States is 100 years of age or older. This prevalence is quickly changing, however, and it is likely that most industrialized nations will soon experience twice that prevalence, or one centenarian per 5000 persons. The ability to survive to extreme old age appears to be the result of a complex combination of genetics, environment, lifestyle, and luck. Understanding the genetics of the very old, and identifying the molecular drivers of longevity (or of mortality), is a potentially powerful approach to discovering and targeting the pathways mediating aging and disease susceptibility and developing preventive and therapeutic agents that will allow more of the population to age in good health.

We have surprisingly found that hydroxytyrosol is able to increase life expectancy in the model of a lower organism, *Drosophila Melanogaster* (fruit fly). The drosophila has been used extensively in gerontological research as a model organism to study treatments that could extend lifespan. It has a short lifespan that allows the efficient study of the effects of products on longevity but nevertheless contains a complex organ and endocrine system.

Preferably hydroxytyrosol is the only active life prolonging ingredient in the composition.

Hydroxytyrosol (3,4-dihydroxyphenylethanol) may be of synthetic origin or it may be obtained together with other water-soluble polyphenols such as tyrosol and oleuropein from extraction of olive leaves, olive fruits and vegetation water of olive oil production.

Thus, the term "hydroxytyrosol" also encompasses any material or extract of a plant containing it, especially in an amount of at least 1.5 weight-%, preferably in an amount of at least 30 weight-%, more preferably in an amount of at least 40 weight-%, most preferably in an amount of at least 50 weight-%, based on the total weight of the plant material or extract. The terms "material of a plant" and "plant material" used in the context of the present invention mean any part of a plant.

In further embodiments of the present invention also hydroxytyrosol derivatives such as esters and physiologically/pharmaceutically acceptable salts may be used instead of hydroxytyrosol. It is also possible to use a mixture of hydroxytyrosol and hydroxytyrosol derivatives.

Derivatives may be e.g. esters. Preferred esters of hydroxytyrosol are e.g. acetates or gucuronide conjugates. The most preferred example of an ester of hydroxytyrosol is oleuropein.

Examples of references that deal with the extraction of oleuropein and/or hydroxytyrosol from olive leaves are WO02/18310, US 2002/0198415, WO2004/005228, US 6,416,808 and US 2002/0058078 which disclose a method for acidic hydrolysis of olive vegetation water for 2 to 12 months until at least 90% of the present oleuropein has been converted. A method of extraction of phenolic compounds from olives, olive pulps, olive oil and oil mill waste water is described by Usana Inc. patents US 6,361,803 and WO01/45514 and in US 2002/0004077. EP-A 1 582 512 describes an extraction of hydroxytyrosol from olive leaves. A method for obtaining hydroxytyrosol and/or oleuropein from the vegetation water of de-pitted olives is disclosed in US 2004/0039066 A1 in paragraphs [0080]-[0091].

The vegetation water may especially have been manufactured according to one of the processes disclosed in US 6,416,808 (column 4, line 37 to column 7, line 27); WO 2004/005228; US 6,936,287; US 2005-103 711; US 2003-108 651; US 2002-198 415; US 6,165,475; JP 2001-252 054; JP 2000-319 161; WO 01/45514 (Usana); US 6,358,542 (see especially column 4, line 1 to column 9, line 50 and examples 1-5 and 11-13); US 6,361,803 (see especially column 3, line 64 to column 9, line 47 and examples 1-5 and 11-13); and WO 2006/084 658.

The vegetation water was preferably manufactured as disclosed in US 6,416,808 (column 4, line 37 to column 7, line 27).

Instead of hydroxytyrosol also a vegetation water concentrate may be used; the use of hydroxytyrosol in a purity of at least 1.5 weight %, preferably of at least 30 weight %, more preferably of at least 50 weight %, is however preferred.

An especially suitable vegetation water concentrate is e.g. "HIDROX® 6%", commercially available from Cre-Agri, Hayward, USA. "HIDROX® 6%" contains 5 to 8 weight-% of proteins, 45 to 68 weight-% of carbohydrates, 17 to 30 weight-% of fat, 8 to 15 weight-% of ash and a minimum of 6 weight-% of water-soluble simple and polyphenols (of these approximately. 2.5 weight-% of hydroxytyrosol), based on the total weight of HIDROX® 6%.

"HIDROX® 2%" and "HIDROX® 9%", both also commercially available from CreAgri, Hayward, USA, may also be used, as well as the following products commercially available from Glanbia and Indena (Milan, Italy): OLNACTIV™ containing from 20 to 35 weight-% of hydroxytyrosol and from 4 to 6 weight-% of tyrosol; OLEASELECT™ having a total content of phenols of $\geq$ 30 weight-% (measured by UV) and an amount of hydroxytyrosol of $\geq$ 1.5 weight-% (measured by HPLC) and an amount of verbascoside of $\geq$ 5.0 weight-% (measured by HPLC) and OLIVE(OLEA)DRY, a powder containing from 22 to 24 g of hydroxytyrosol and from 5.0 to 6.5 g of tyrosol per kg.

Further suitable products are Prolivols, commercially available from Seppic, containing 35 weight-% of polyphenols, especially 20 mg hydroxytyrosol (per g of Prolivols) and 3 mg of tyrosol (per g of Prolivols); as well as Olive Braun Standard 500 (from obipektin): a powder containing from 1.0 to 2.2 g of hydroxytyrosol and from 0.2 to 0.7 g of tyrosol per kg; Olivex olive polyphenol liquid P10 (from Albert Isliker): a liquid containing from 2.0 to 3.5 g of hydroxytyrosol and from 0.2 to 1.0 g of tyrosol per kg; Olivex olive polyphenol (from Albert Isliker): a powder containing from 22 to 23 g of hydroxytyrosol and from 6.5 to 8.0 g of tyrosol per kg; and Olive Polyphenols NLT (from Lalilab Inc.) containing from 2.0 to 6 weight-% of hydroxytyrosol and from 0.7 to 1.1 weight-% of tyrosol.

In suitable commercially available vegetation water concentrates the amount of hydroxytyrosol varies in the range of from 1.0 to 220 g per kg of the total weight of the vegetation water concentrate. The amount of tyrosol preferably varies in the range of from 0.2 to 45 g per kg of the total weight of the vegetation water concentrate. The weight ratio of hydroxytyrosol to tyrosol is preferably between 100 : 10 and 100 : 40, most preferably between 100 : 18 and 100 : 35.

The vegetation water is also commercially known as "olive juice", the dried products obtained thereof also as "olive juice powder" or "olive juice preparations".

"Essentially not comprising resveratrol" means that the amount of resveratrol in the composition is $\leq$ 1 weight-%, preferably $\leq$ 0.5 weight-%, more preferably $\leq$ 0.1 weight-%, based on the total weight of the composition. It also means that resveratrol is not added intentionally to the composition. Resveratrol may only be in the composition as by-product of a hydroxytyrosol extract/concentrate obtained from plants or fruit of plants such as olives.

Preferably the composition is administered orally to animals, which means that the composition is in any form that can be eaten or drunk by animals or put into the stomach of animals via the mouth/jaw.

Thus, the composition is preferably selected from the group of dietary supplements, food additives, functional food, feed additives, functional feed, food premixes, feed premixes, and beverages.

Examples of forms of dietary supplements are tablets, pills, granules, dragées, capsules, instant drinks and effervescent formulations.

Examples of food/feed additives are any composition/formulation added to food/feed during its manufacture or its preparation for consumption.

Examples of functional food are dairy products (yoghurts), cereal bars and bakery items such as cakes, cookies, and bread. Clinical nutrition is also encompassed.

Examples of functional feed including pet food compositions are feed intended to supply necessary dietary requirements, as well as treats (e.g., dog biscuits) or other feed supplements. The animal feed comprising the composition according to the invention may be in the form of a dry composition (for example, kibble), semi-moist composition, wet composition, or any mixture thereof. Alternatively or additionally, the animal feed is a supplement, such as a gravy, drinking water, yogurt, powder, suspension, chew, treat (e.g., biscuits) or any other delivery form.

Examples of food premixes are premixes for manufacture of dairy products, cereal bars, and bakery items such as cakes and cookies, and soups.

A further aspect of the invention relates to a feed additive or additive composition, such as to be added to one or more edible feed substance (s) or ingredient (s), for example to prepare a feed composition or for supplementation to an existing feed to form a feed composition.

The so-called premixes are examples of animal feed additives of the invention. A premix designates a preferably uniform mixture of one or more micro-ingredients with diluent and/or carrier. Premixes are used to facilitate uniform dispersion of micro-ingredients in a larger mix.

The premix may be in the form of granules or pellets.

In a particular embodiment, hydroxytyrosol, in the form in which it is added to the feed, or when being included in a feed additive, is well-defined. The term well-defined means that the hydroxytyrosol preparation is at least 30% pure. In other particular embodiments the well-defined hydroxytyrosol preparation is at least 50, 60, 70, 80, 85, 88, 90, 92, 94, or at least 95% pure.

Usually fat- and water-soluble vitamins, as well as trace minerals form part of a so-called premix intended for addition to the feed, whereas macro minerals are usually separately added to the feed.

Further, optional, feed-additive ingredients are coloring agents, e.g. carotenoids such as beta-carotene, astaxanthin, and lutein; aroma compounds; stabilisers; antimicrobial peptides; reactive oxygen generating species; and/or at least one enzyme selected from amongst phytase (EC 3.1.3.8 or 3.1.3.26); xylanase (EC 3.2.1.8); galactanase (EC 3.2.1.89); alpha-galactosidase (EC 3.2.1.22); protease (EC 3.4., phospholipase A1 (EC 3.1.1.32); phospholipase A2 (EC 3.1.1.4); lysophospholipase (EC 3.1.1.5); phospholipase C (EC 3.1.4.3); phospholipase D (EC 3.1.4.4); amylase such as, for example, alpha-amylase (EC 3.2.1.1); and/or beta-glucanase (EC 3.2.1.4 or EC 3.2.1.6).

Beverages encompass non-alcoholic and alcoholic drinks as well as liquid preparations to be added to drinking water and liquid food. Non-alcoholic drinks are e.g. instant drinks, soft drinks, sport drinks or sport beverages in general, fruit juices such as e.g. orange juice, apple juice and grapefruit juice; vegetable juices such as tomato juice; smoothies, lemonades, functional water, near-water drinks (i.e. water based drinks with a low calorie content), teas and milk based drinks. Alcoholic drinks are especially beer. Liquid food are e.g. soups and dairy products (e.g. muesli drinks).

The dietary supplements according to the present invention may further contain protective hydrocolloids, binders, film forming agents, encapsulating agents/materials, wall/shell materials, matrix compounds, coatings, emulsifiers, surface active agents, solubilizing agents (oils, fats, waxes, lecithins etc.), adsorbents, carriers, fillers, co-compounds, dispersing agents, wetting agents, processing aids (solvents), flowing agents, taste masking agents, weighting agents, jellyfying agents, gel forming agents, antioxidants and antimicrobials.

Alternatives to dietary supplements which may also be used and are encompassed by the present invention are pharmaceutical compositions or OTC (over the counter) preparations.

Beside a pharmaceutically acceptable carrier and hydroxytyrosol (derivatives) with the preferred purity (and further preferences) as given above, the pharmaceutical compositions according to the present invention may further contain conventional pharmaceutical additives and adjuvants, excipients or diluents, including, but not limited to, water, gelatin of any origin, vegetable gums, ligninsulfonate, talc, sugars, starch, gum arabic, vegetable oils, polyalkylene glycols, flavoring agents, preservatives, stabilizers, emulsifying agents, buffers, lubricants, colorants, wetting agents, fillers, and the like. The carrier material can be organic or inorganic inert carrier material suitable for oral administration.

The dietary supplements and the pharmaceutical compositions according to the present invention may be in any galenic form that is suitable for oral administration to the animal body (preferably the human body), e.g. in solid form such as tablets, pills, granules, dragées, capsules, and effervescent formulations such as powders and tablets, or in liquid form such as solutions, emulsions or suspensions as e.g. beverages, pastes and oily suspensions. The pastes may be filled into hard or soft shell capsules. The dietary and pharmaceutical compositions may be in the form of controlled (delayed) release formulations.

According to the present invention such compositions are used for prolonging the life of animals.

Thus, the present invention is also directed to a composition (with the forms and preferences as given above) which is administered to animals (preferably humans) comprising hydroxytyrosol for prolonging the life of said animals (preferably humans), wherein the composition does essentially not comprise resveratrol. Preferably the composition is administered orally.

Furthermore, the present invention is directed to a method of prolonging the life of an animal by administering to said animal an effective amount of hydroxytyrosol or an effective amount of a composition comprising hydroxytyrosol, wherein the composition does essentially not comprise resveratrol.

Animals in the context of the present invention include humans and encompass mammals, fish and birds. Preferred "animals" are humans, pet animals and farm animals as well as camels and elephants, whereas humans are especially preferred.

Examples for pet animals are dogs, cats, birds, toy fish, guinea pigs, hamsters, rabbits, and ferrets. Examples for farm animals are fish, pigs, horses, ruminants (cattle, sheep and goat) and poultry.

"Prolonging the life of an animal" in the context of the present invention means

reducing the risk to die at a certain age compared to untreated;
increasing the chance to live longer (longevity), i.e. increasing the average life expectancy compared to untreated; and/or
extending the lifetime independent of medical care.

Further definitions that are also encompassed by the present invention are given by Hermann Brenner and Volker Arndt in Experimental Gerontoloy 2004, 39, 679-686 : "Epidemiology in aging research".

The starting point for calculating life expectancies is the age-specific death rates of the population members. For example, if 10% of a group of people alive at their 90th birthday die before their 91 st birthday, then the age-specific death rate at age 90 would be 10%.

These values are then used to calculate a life table, from which one can calculate the probability of surviving to each age. In actuarial notation the probability of surviving from age x to age x+n is denoted $_nP_x$ and the probability of dying during age x (i.e. between ages x and x+1) is denoted $q_x$.

The life expectancy at age x, denoted $e_x$, is then calculated by adding up the probabilities to survive to every age. This is the expected number of complete years lived (one may think of it as the number of birthdays they celebrate).

$$e_x = \sum_{t=1}^{\infty} {}_t p_x = \sum_{t=0}^{\infty} t \, {}_t p_x q_{x+t}$$

Because age is rounded down to the last birthday, on average people live half a year beyond their final birthday, so half a year is added to the life expectancy to calculate the full life expectancy.

Life expectancy is by definition an arithmetic mean. It can be calculated also by integrating the survival curve from ages 0 to positive infinity (the maximum lifespan, sometimes called 'omega'). For an extinct cohort (all people born in year 1850, for example), of course, it can simply be calculated by averaging the ages at death. For cohorts with some survivors it is estimated by using mortality experience in recent years.

Note that no allowance has been made in this calculation for expected changes in life expectancy in the future. Usually when life expectancy figures are quoted, they have been calculated like this with no allowance for expected future changes. This means that quoted life expectancy figures are not generally appropriate for calculating how long any given individual of a particular age is expected to live, as they effectively assume that current death rates will be "frozen" and not change in the future. Instead, life expectancy figures can be thought of as a useful statistic to summarize the current health status of a population. Some models do exist to account for the evolution of mortality (e.g., the Lee-Carter model as disclosed by Ronald D. Lee and Lawrence Carter in Journal of the American Statistical Association 1992 (September), 87, 659-671: "Modeling and Forecasting the Time Series of U.S. Mortality".

The use of the fruit fly to determine the longevity in other species, including humans is e.g. disclosed in American Journal of Medicine 2004, 117(11), 851-860: "The genetics of human longevity" by Browner WS, Kahn AJ, Ziv E, Reiner AP, Oshima J, Cawthon RM, Hsueh WC, and Cummings SR. The authors of this scientific article found out that "Many of the genes that affect aging and longevity in model organisms, such as mice, fruit flies, and worms, have human homologs."

The drosophila longevity model responds positively to dietary restriction (DR). Dietary restriction by undernutrition without malnutrition, i.e. the restricted diets are packed with enough protein, fat, vitamins and minerals to avoid any deficiency in essential nutrients and maintain the animal's body function, is known to increase the longevity/ lifespan in a number of aging models such as yeast, nematodes, fruit flies and mice. Typically, test organisms in dietary-restriction studies consume a diet of 30 to 50 percent fewer calories than their free-eating counterparts. These restricted diets are packed with enough protein, fat, vitamins and minerals to maintain the animal's body function. DR consistently increased the lifespan of several mammals (mice, rats, guinea pigs). Recent data suggest that dietary restriction may extend the lifespan of monkeys and possibly humans. Finally, a number of studies showed that pathways involved in regulating lifespan are conserved through evolution and across species as diverse as nematodes and humans. This is the case for key molecular pathways involved in lifespan regulation such as the insulin signaling pathways, which is conserved from the unicellular yeasts to mammals. The insulin signaling pathway has been shown to play a critical role in the regulation of lifespan and also of growth and size in different species. This conservation indicates that certain physiological processes affecting life span are conserved across species and strongly suggests that treatments increasing the longevity of simple animals are likely to be important for mammalian longevity. Thus, Drosophila has become an accepted model to identify compounds that could prolong longevity in humans.

The daily dosage of hydroxytyrosol for humans (70 kg person) may be at least 0.1 mg. It may vary from 5 to 500 mg, preferably from 15 to 100 mg.

The preferred dose of hydroxytyrosol varies from 0.28 to 1.9 mg/kg metabolic body weight for mammals, whereby

$$\text{``metabolic body weight'' [in kg]} = (\text{body weight [in kg]})^{0.75}$$

for mammals. That means e.g. that for a human of 70 kg the preferred daily dose would vary between 6.77 and 45.98 mg, for a 20 kg dog the preferred daily dose would vary between 2.23 and 15.1 mg.

The invention is now further illustrated by the following, non-limiting examples.

**Examples**

Example 1: Soft gelatin capsule

Soft gelatin capsules are prepared by conventional procedures providing a dose of hydroxytyrosol of 50 mg per capsule. A suitable daily dose is 1 to 5 capsules.

Other ingredients: glycerol. Water, gelatine, vegetable oil

Example 2: Hard gelatin capsule

Hard gelatin capsules are prepared by conventional procedures providing a dose of hydroxytyrosol of 75 mg per capsule. A suitable daily dose is 1 to 5 capsules.

Other ingredients:

Fillers: lactose or cellulose or cellulose derivatives q.s.
Lubricant: magnesium stearate if necessary (0.5%)

Example 3: Tablet

Tablets are prepared by conventional procedures providing as active ingredient 100 mg of hydroxytyrosol per tablet, and as excipients microcrystalline cellulose, silicone dioxide ($SiO_2$), magnesium stearate, crosscarmellose sodium ad 500 mg.

Example 4: soft drink

A soft drink containing hydroxytyrosol may be prepared as follows:

| ingredient | [g] |
|---|---|
| **A.** juice concentrates and water soluble flavours | |
| 60.3°Brix, 5.15% acidity | 657.99 |
| 43.5° Brix, 32.7% acidity | 95.96 |
| Orange flavour, water soluble | 3.43 |
| Apricot flavour, water soluble | 6.71 |
| water | 26.46 |
| **B.** color | |
| β-carotene 10% CWS | 0.89 |
| water | 67.65 |
| **C.** Acid and antioxidant | |
| Ascorbic acid | 4.11 |
| Citric acid anhydrous | 0.69 |
| water | 43.18 |
| **D.** stabilizers | |
| pectin | 0.20 |
| Sodium benzoate | 2.74 |
| water | 65.60 |

(continued)

| ingredient | [g] |
|---|---|
| **E.** oil soluble flavours | |
| Orange flavour, oil soluble | 0.34 |
| Orange oil distilled | 0.34 |
| **F.** active ingredient | |
| Hydroxytyrosol | Amount providing 15 mg |

Fruit juice concentrates and water soluble flavours are mixed without incorporation of air. The color is dissolved in deionized water. Ascorbic acid and citric acid are dissolved in water. Sodium benzoate is dissolved in water. The pectin is added under stirring and dissolved while boiling. The solution is cooled down. Orange oil and oil soluble flavours are premixed. The active ingredient as mentioned under F is stirred into the fruit juice concentrate mixture of A.

In order to prepare the soft drinks all components A-F are mixed together before homogenizing using a Turrax and then a high-pressure homogenizer ($p_1$ = 200 bar, $p_2$ = 50 bar).

Example 5: Life extending experiments with Drosophila

*Drosophila melanogaster (Oregon* wild strain) were used for lifespan study. Flies were reared in a light/dark cycle of 12:12 hours at a temperature of 25°C and 65% relative humidity. *Drosophila* were fed with a standard medium consisting of 170 g of corn flour, 15 g of agar, 15 g of yeast, 10 ml of 100% propionic acid, 130 g of sucrose, and 2000 ml of water.

Lifespan studies

One-day-old *Drosophila* were transferred to plastic vials (25x100mm) containing 2 ml of the diet medium, which was replaced every 3 days. 10 flies from each gender were kept in one vial. Hydroxytyrosol was provided in the diet to *Drosophila* from age of 15 days on. The *Drosophilae* were counted every 3 days until all flies died. Survivals were calculated at the end of the experiments after correcting total loss incurred from handling (Phillips JP, Campbell SD, Michaud D, Charbonneau M, Hilliker AJ, Proc Natl Acad Sci U S A. 1989, 86(8), 2761-2765: "Null mutation of copper/ zinc superoxide dismutase in Drosophila confers hypersensitivity to paraquat and reduced longevity."). The mean lifespan was calculated according to the method described by Cui X, Dai XG, Li WB, Zhang BL, Fang YZ in Am. J. Chin. Med. 1999, 27(3-4), 407-413: "Effects of lu-duo-wei capsule on prolonging life span of housefly and Drosophila melanogaster.".

**Results**

**Hydroxytorosol** was tested at 1, 5, 10, 20, and 50 mg/100 g in the diet. All concentrations extended the lifespan in male *Drosophila* with best effect seen at 5 mg/100 g diet (11.5%).

| | Control | Hydroxytyrosol 1mg/ 100g | Hydroxytyrosol 5mg/ 100g | Hydroxytyrosol 10mg/ 100g | Hydroxytyrosol 20mg/ 100g | Hydroxytyrosol 50mg/ 100g |
|---|---|---|---|---|---|---|
| **Total number of Drosophila** | 299 | 264 | 253 | 290 | 300 | 300 |
| **Mean Lifespan (day)** | 69.42 | 75.14 | 77.44 | 72.12 | 74.26 | 72.21 |
| **STDEV (day)** | 17.83 | 34.71 | 19.23 | 20.53 | 17.16 | 18.71 |
| **%Increase of lifespan compared to control** | | 8.23 | 11.55 | 3.89 | 6.97 | 4.02 |

**Claims**

1. Use of a composition comprising hydroxytyrosol as life prolonging agent, wherein the composition does essentially not comprise resveratrol.

2. The use according to claim 1, wherein hydroxytyrosol is the only active life prolonging ingredient in the composition.

3. The use according to claim 1 or 2, wherein the composition is administered orally to animals.

4. The use according to claim 3, wherein the composition is selected from the group of dietary supplements, food additives, functional food, food premixes, feed additives, functional feed, feed premixes, and beverages.

5. Composition for animals comprising hydroxytyrosol for reducing the risk to die at a certain age compared to animals not treated with said composition; for increasing the chance to live longer (longevity), i.e. for increasing the average life expectancy compared to animals not treated with said composition; and/or for extending the lifetime independent of medical care, wherein the composition does essentially not comprise resveratrol.

6. The composition according to claim 5, wherein the composition is administered orally to said animals.

7. The composition according to claim 6, wherein the composition is in form of a dietary supplement, a food additive, a functional food, a feed additive, a functional feed or a beverage.

8. The composition according to any one or more of claims 5 to 7, wherein the animals are humans.

9. Method of prolonging the life of animals by administering to said animal an effective amount of hydroxytyrosol or an effective amount of a composition comprising hydroxytyrosol, wherein the composition does essentially not comprise resveratrol.

10. Method of reducing the risk to die at a certain age; of increasing the chance to live longer (longevity), i.e. of increasing the average life expectancy; and/or of extending the lifetime independent of medical care by administering to said animal an effective amount of hydroxytyrosol or an effective amount of a composition comprising hydroxytyrosol, wherein the composition does essentially not comprise resveratrol.

**European Patent Office**

**PARTIAL EUROPEAN SEARCH REPORT**

which under Rule 45 of the European Patent Convention shall be considered, for the purposes of subsequent proceedings, as the European search report

Application Number

EP 07 00 7874

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | PEREZ-JIMENEZ F ET AL: "International conference on the healthy effect of virgin olive oil." EUROPEAN JOURNAL OF CLINICAL INVESTIGATION JUL 2005, vol. 35, no. 7, July 2005 (2005-07), pages 421-424, XP002452265 ISSN: 0014-2972 * page 422, paragraphs 2,3 * * page 423, paragraphs 3,4 * | 1,3-10 | INV. A23L1/30 A61K31/05 A61K31/7048 A23L2/52 |
| X | WO 2004/110171 A (NATRACEUTICAL S A [ES]; ZUMBE ALBERT [CH]) 23 December 2004 (2004-12-23) * page 8, lines 3-17; claims 1,21 * * page 1, lines 6-25 * | 1,3-10 | |
| X | JP 2001 181632 A (NISSHIN OIL MILLS LTD) 3 July 2001 (2001-07-03) * abstract * | 1,3-10 | |
| X | JP 2002 153238 A (NIPPON SHINYAKU CO LTD) 28 May 2002 (2002-05-28) * abstract * | 1,3-10 | **TECHNICAL FIELDS SEARCHED (IPC)** A23L A61K |

-/--

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| The Hague | 25 September 2007 | Tallgren, Antti |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C07)

European Patent

Office

**PARTIAL EUROPEAN SEARCH REPORT**

Application Number

EP 07 00 7874

| | DOCUMENTS CONSIDERED TO BE RELEVANT | | CLASSIFICATION OF THE APPLICATION (IPC) |
|---|---|---|---|
| Category | Citation of document with indication, where appropriate, of relevant passages | Relevant to claim | |
| X | WO 03/068171 A (CREAGRI INC [US]) 21 August 2003 (2003-08-21) * page 3, lines 16-29; claims 1,4,16 * * page 18, line 25 - page 19, line 7 * ----- | 1-10 | |
| X | VISIOLI FRANCESCO ET AL: "Local food and cardioprotection: the role of phytochemicals." FORUM OF NUTRITION 2006, vol. 59, 2006, pages 116-129, XP008084087 ISSN: 1660-0347 * page 123, paragraph 4 - page 124, paragraph 2 * ----- | 1,3-10 | |
| D,A | WO 2004/005228 A (CREAGRI INC [US]; CREA ROBERTO [US]) 15 January 2004 (2004-01-15) * claims 1,24 * ----- | 1-10 | TECHNICAL FIELDS SEARCHED (IPC) |

EPO FORM 1503 03.82 (P04C10)

**European Patent Office**

**INCOMPLETE SEARCH**
**SHEET C**

Application Number

EP 07 00 7874

Although claims 9 and 10 are directed to a method of treatment of the human/animal body (Article 52(4) EPC), the search has been carried out and based on the alleged effects of the compound/composition.

- - - - -

Claim(s) searched completely:
        1-8

Claim(s) searched incompletely:
        9,10

Reason for the limitation of the search (non-patentable invention(s)):

Article 52 (4) EPC - Method for treatment of the human or animal body by therapy

## ANNEX TO THE EUROPEAN SEARCH REPORT
## ON EUROPEAN PATENT APPLICATION NO.

EP 07 00 7874

This annex lists the patent family members relating to the patent documents cited in the above-mentioned European search report.
The members are as contained in the European Patent Office EDP file on
The European Patent Office is in no way liable for these particulars which are merely given for the purpose of information.

25-09-2007

| Patent document cited in search report | | Publication date | Patent family member(s) | | Publication date |
|---|---|---|---|---|---|
| WO 2004110171 | A | 23-12-2004 | AT<br>EP<br>ES | 349914 T<br>1633204 A2<br>2280029 T3 | 15-01-2007<br>15-03-2006<br>01-09-2007 |
| JP 2001181632 | A | 03-07-2001 | NONE | | |
| JP 2002153238 | A | 28-05-2002 | NONE | | |
| WO 03068171 | A | 21-08-2003 | AU<br>CA<br>CN<br>EP<br>JP<br>NZ | 2003211118 A1<br>2474798 A1<br>1646093 A<br>1482909 A2<br>2005517033 T<br>534884 A | 04-09-2003<br>21-08-2003<br>27-07-2005<br>08-12-2004<br>09-06-2005<br>31-05-2007 |
| WO 2004005228 | A | 15-01-2004 | AU<br>CA<br>CN<br>EP<br>JP<br>KR | 2003249719 A1<br>2491613 A1<br>1665764 A<br>1523465 A1<br>2005532398 T<br>20050025588 A | 23-01-2004<br>15-01-2004<br>07-09-2005<br>20-04-2005<br>27-10-2005<br>14-03-2005 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0218310 A
- US 20020198415 A
- WO 2004005228 A
- US 6416808 B
- US 20020058078 A
- US 6361803 B
- WO 0145514 A
- US 20020004077 A
- EP 1582512 A
- US 20040039066 A1

- US 6936287 B
- US 2005103711 A
- US 2003108651 A
- US 2002198415 A
- US 6165475 A
- JP 2001252054 A
- JP 2000319161 A
- US 6358542 B
- WO 2006084658 A

**Non-patent literature cited in the description**

- *the American Journal of Clinical Nutrition,* February 2006, vol. 83 (2), 484S-487S
- **HERMANN BRENNER ; VOLKER ARNDT.** *Experimental Gerontoloy,* 2004, vol. 39, 679-686
- **RONALD D. LEE ; LAWRENCE CARTER.** *Journal of the American Statistical Association,* September 1992, vol. 87, 659-671
- *American Journal of Medicine,* 2004, vol. 117 (11), 851-860

- **PHILLIPS JP ; CAMPBELL SD ; MICHAUD D ; CHARBONNEAU M ; HILLIKER AJ.** Null mutation of copper/zinc superoxide dismutase in Drosophila confers hypersensitivity to paraquat and reduced longevity. *Proc Natl Acad Sci U S A.,* 1989, vol. 86 (8), 2761-2765
- **CUI X ; DAI XG ; LI WB ; ZHANG BL ; FANG YZ.** Effects of lu-duo-wei capsule on prolonging life span of housefly and Drosophila melanogaster. *Am. J. Chin. Med.,* 1999, vol. 27 (3-4), 407-413